# EUROPEAN PATENT APPLICATION

(11) **EP 3 872 752 A1**
(43) Date of publication of application: **01.09.2021**
(21) Application number: 20159930.5
(22) Date of filing: 27.02.2020
(51) Int. Cl.: G06T 5/50, A61B 6/00

(54) **MEDICAL IMAGE DATA**

(71) Applicant: Siemens Healthcare GmbH, 91052 Erlangen (DE)
(72) Inventor: SUDARSKY, Sandra, Bedminster, NJ New Jersey 07921 (US)
(74) Representative: EIP

(57) **Abstract**

Disclosed is a method, a computer readable storage medium and a system for processing angiography data. Angiography data is received at a data processing system. The angiography data is collected by scanning an imaged volume of a patient into whose vessels a contrast agent has been introduced. Data indicating a user-designated region of interest within the imaged volume is received at the data processing system. A determination process is performed at the data processing system. The determination process acts on the angiography data to determine at least one regional characteristic, which is indicative of the concentration of the contrast agent within the region of interest. A visualization process is performed at the data processing system. The visualization process acts on the angiography data and the at least one regional characteristic, to generate image data representative of a view of the region of interest.

## Description

### Technical Field

The present invention relates to processing medical image data, in particular angiography data, using a data processing system.

### Background

Angiography may be defined as a class of medical imaging techniques that are used to obtain information regarding the circulatory system of a human or animal body. The circulatory system includes the cardiovascular system and the lymphatic system. Thus, angiography might be used to study the the arteries, veins and/or heart of a patient's cardiovascular system or might be used to study the lymphatic vessels of a patient's lymphatic system.

Angiography may be carried out with various types of medical imaging apparatus, such as X-ray imaging apparatus (and, in particular examples, with an X-ray computed tomography apparatus), or magnetic resonance apparatus.

In many cases, when carrying out angiography, a contrast agent is introduced into the patient in order to increase the visibility of the parts of the circulatory system that are of interest. The introduction of the contrast agent is usually achieved by means of injection or infusion.

Certain classes of contrast agents may be particularly suited to use with certain types of imaging apparatus. For instance, where an X-ray imaging apparatus is used, a radiocontrast agent may be used, which may, for example contain a radiopaque material, such as an iodine or barium containing material. Where a magnetic resonance imaging apparatus is used, a paramagnetic material may be used, such as a gadolinium containing material.

In many cases, angiography provides a medical professional with an angiogram, a visual representation of the circulatory system (or parts thereof). However, such visual representations, may not reliably show the medical professional the circulatory system with a satisfactory level of clarity and/or intelligibility.

It is an object of the present invention to address at least some of the difficulties with the procedure for analysing medical image data.

### Summary

According to a first aspect of the present invention, there is provided a method for processing angiography data. The method comprises: receiving, at a data processing system, angiography data, which is collected by scanning an imaged volume of a patient into whose vessels a contrast agent has been introduced; receiving, at the data processing system, data indicating a user-designated region of interest within the imaged volume; performing, at the data processing system, a determination process, which acts on the angiography data to determine at least one regional characteristic, which is indicative of the concentration of the contrast agent within the region of interest; performing, at the data processing system, a visualization process, which acts on the angiography data and the at least one regional characteristic, to generate image data representative of a view of the region of interest.

The concentration of contrast agent is found to vary throughout the body of the patient. For example, the concentration of contrast agent may tend to be greater near to the site where the contrast agent was introduced (e.g. by infusion or injection). Additionally, the concentration of contrast agent at a given location within the body may change over time, for instance as a result of the contrast agent flowing through the circulatory system.

Such variations in the concentration of contrast agent tend to complicate the task of providing a user with a clear and easily intelligible visual representation of the circulatory system. A visualization process that provides the user with a view of the entire imaged area of the patient would need to adjust for the considerable variations in contrast agent throughout the imaged area. In order to provide the user with a clear visual representation, such adjustments for differing contrast agent concentrations would need to be applied smoothly; otherwise, visual artefacts might appear in the view provided to the user. However, reliably achieving such smooth adjustments presents significant challenges.

In methods according to the first aspect, visualization is carried out for a user-designated region of interest, with a regional characteristic that is indicative of the concentration of the contrast agent within the region of interest. The inventor reasons that variations in the concentration of the contrast agent will tend to be smaller within a region of interest than in the full area/volume imaged. This is because, firstly, the region of interest is physically smaller than the imaged volume. Secondly, this is because a region of interest may be expected to include contiguous portions of the circulatory system and, given that contrast agent is diluted as it travels through the circulatory system, such contiguous portions may be expected to have similar concentrations of contrast agent. Thus, fewer issues are expected to arise when generating a visual representation of the angiographic data using a regional characteristic that is indicative of the concentration of the contrast agent within the region of interest.

In particular examples, the data indicating the region of interest may comprise user view direction data, for example gaze direction data. It is considered that this makes the angiographic data particularly intelligible to the user. It is expected that users will intuitively understand the adjustments to the visualization process as their gaze direction changes. In specific examples, as the gaze direction changes, the visualization adjustment transitions smoothly. Additionally, if the user's field of view is occupied by a view of the region of interest, it may be of less importance to the user how the areas of the imaged volume outside of the region of interest are visualized. Indeed, in some embodiments, areas of the imaged volume outside of the region of interest may not be visualized

In examples, the region of interest may have a predetermined maximum size, for instance defined in absolute terms. For example, its largest dimension may be 40cm, in some cases 30cm, or in other cases 20cm.

In some examples, the determination process may simply comprise analysing an overall distribution, for the region of interest, of values of a measured variable that is represented within the angiographic data. For example, the determination process might determine the most common value for the measured variable for the full distribution (i.e. the mode), or for some sub-range of the values for the measured variable that is associated with the contrast agent.

In other examples, the determination process may comprise analysing spatial patterns within a subset of the angiography data that corresponds to the region of interest. For one example, the determination process may comprise identifying vessel-like forms within the region of interest. For instance, simple geometric forms, such as cylinders and truncated cones, may be identified. In another example, a segmentation process may be used, which may for example utilize a neural network. In particular examples, a deep learning neural network may be used.

According to a second aspect of the present invention, there is provided a computer readable storage medium, storing instructions that, when executed by a processor, cause the processor to: receive angiography data, which is collected by scanning an imaged volume of a patient into whose vessels a contrast agent has been introduced; receive data indicating a user-designated region of interest within the imaged volume; perform a determination process, which acts on the angiography data to determine at least one regional characteristic, which is indicative of the concentration of the contrast agent within the region of interest; and perform a visualization process, which acts on the angiography data and the at least one regional characteristic, to generate image data representative of a view of the region of interest.

Providing a computer readable storage medium allows the advantageous method according to the first aspect to be used, for example, by medical professionals. The computer readable storage medium allows access to the method according to the first aspect which has the above described advantages.

According to a third aspect of the present invention, there is provided a system for processing medical image data, comprising: one or more processors; and a memory storing instructions that, when executed by the one or more processors, cause the one or more processors to: receive angiography data, which is collected by scanning an imaged volume of a patient into whose vessels a contrast agent has been introduced; receive data indicating a user-designated region of interest within the imaged volume; perform a determination process, which acts on the angiography data to determine at least one regional characteristic, which is indicative of the concentration of the contrast agent within the region of interest; and perform a visualization process, which acts on the angiography data and the at least one regional characteristic, to generate image data representative of a view of the region of interest.

The third aspect advantageously provides hardware for implementing the method according to the first aspect, which has the above-described advantages.

Optionally, the system according to the third aspect comprises a gaze tracking device, which generates data representative of a gaze direction for the user, wherein the data indicating the region of interest comprise the data generated by the gaze tracking device.

It is considered that such a system makes the angiographic data particularly intelligible to the user. It is expected that users will intuitively understand the adjustments to the visualization process as their gaze direction changes. The system may in particular examples be configured such that, as the gaze direction changes, the visualization adjustment transitions smoothly. Additionally, if the user's field of view is occupied by a view of the region of interest, it may be of less importance to the user how the areas of the imaged volume outside of the region of interest are visualized.

Optionally, the system according to the third aspect comprises an imaging apparatus configured to acquire the medical image data.

Incorporating the imaging apparatus advantageously provides a system which can perform the additional task of acquiring the relevant angiographic data.

Optionally, the apparatus according to the third aspect comprises an input interface for allowing a user of the apparatus to override and/or manually correct the output of the apparatus.

The input interface advantageously allows the user to make changes to the output as required. The user can therefore modify the output if the results are judged to be inaccurate, for example. In particular, the user can use the interface to manually adjust the regional characteristic.

### Brief Description of the Drawings

The present invention will now be described, by way of example only, with reference to the accompanying drawings in which:
Figure 1 is a flow diagram illustrating a method for processing angiographic data, according to an example;
Figure 2 is a schematic diagram representing an imaged volume and a region of interest within the anatomy of a patient;
Figures 3A and 3B show histograms for values of a measured variable for a region of interest;
Figures 4A-4B illustrate respective views that are generated by a visualization process acting on the same angiographic data, but different user-designated regions of interest;
Figure 5 is a schematic diagram illustrating a system for processing medical image data according to an example;
Figures 6A and 6B illustrate a system for processing medical image data according to another example, where the system comprises a headset worn by a user.

### Detailed Description

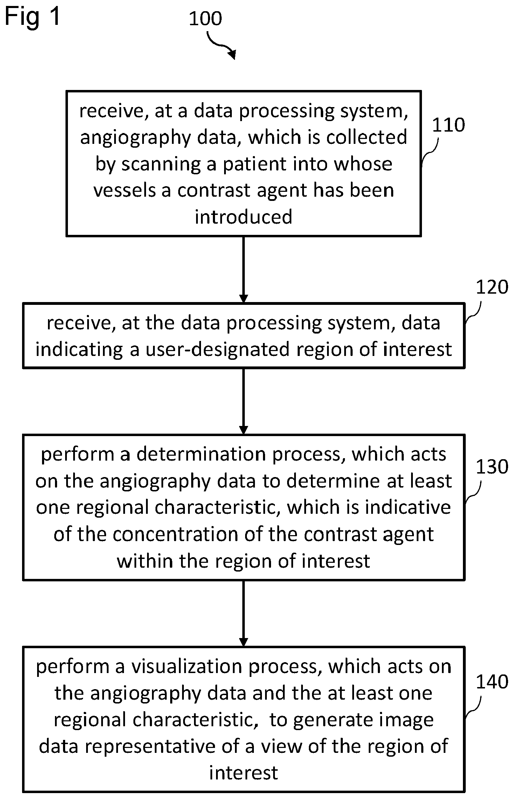

Figure 1 is a block diagram illustrating a method 100 for processing angiographic data. The method 100 is hereafter referred to as the angiographic data processing method 100.

At block 110 of the angiographic data processing method 100, angiography data is received at a data processing system. Examples and further details regarding the data processing systems are provided further below. The angiography data may be X-ray image data, MRI image data, computed tomography scan data, or any other kind of medical image data.
The angiographic data may represent an imaged volume 200 within the human or animal body. Thus, the input medical input data may be said to be three-dimensional data.

The angiography data are collected by scanning a patient into whose vessels a suitable contrast agent has been introduced. As noted above, in general, such a contrast agent is intended to significantly increase the visibility of the parts of the circulatory system in which it is present.

In many cases, the angiographic data comprises a plurality of data elements, each of which includes a value for a measured variable, and corresponds to a one of a plurality of locations that are distributed over the imaging volume 200. Typically, the measured variable will correspond to the type of imaging apparatus used to generate the angiographic data. For example, with angiographic data generated using an X-ray imaging apparatus, the measured variable may relate to radiodensity, for example the measured variable may be attenuation, e.g. measured on the Hounsfield scale. As another example, with angiographic data generated using a magnetic resonance imaging apparatus, the measured variable may be the T₁ time constant or the T₂ time constant. In some cases, each data element within the angiographic data may define the value of the measured variable and the associated location. Furthermore, it is envisaged that in some cases the angiographic data may define, for each location, respective values for two or more measured variables. For example, with angiographic data generated using a magnetic resonance imaging apparatus, the value of both the T₁ time constant and the T₂ time constant may be stored for each location.

The angiographic data may have been acquired using a three-dimensional (3D) acquisition process, so that it is inherently in a 3D format. As an alternative, the angiographic data may have been derived from a set of two dimensional (2D) images in one or more imaging planes, with each of the 2D images being made up of a number of pixels.

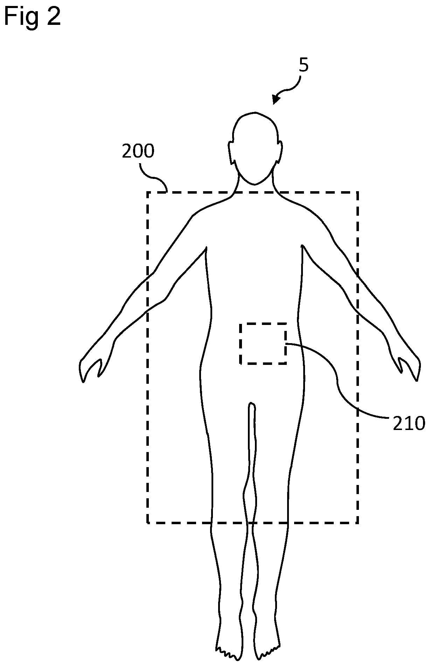

At block 120, data indicating a user-designated region of interest 210 is received at the data processing system. Figure 2 illustrates schematically a region of interest 210 that is located within a larger imaged volume 200 of the body of a patient 5.

In some examples, the data indicating the region of interest may comprise user gaze direction data (e.g. data defining the direction in which the user's eyes are looking) and/or user view direction data (e.g. data defining the direction in which a user's head is facing). It is considered that such an approach may make the angiographic data particularly intelligible to the user, as the visualization of block 140 (discussed below) may react seamlessly and intuitively to the user's eye movements.

In other examples, the data indicating the region of interest might simply be generated by a user input device such as a pointing device (e.g. a mouse, trackpad, or joystick), or a touchscreen).

At block 130 of the angiographic data processing method 100, a determination process is performed at the data processing system. This determination process acts on the angiography data to determine a regional characteristic (or, in some cases, a plurality of regional characteristics) indicative of the concentration of the contrast agent within the region of interest. In particular, the determination process may act on the angiography data corresponding to the region of interest.

In some examples, the determination process may comprise analysing an overall distribution, for the region of interest, of the values of the (or each) measured variable represented within the angiographic data. For example, the determination process might analyse the relative frequency with which the values of the measured variable fall into various sub-ranges.

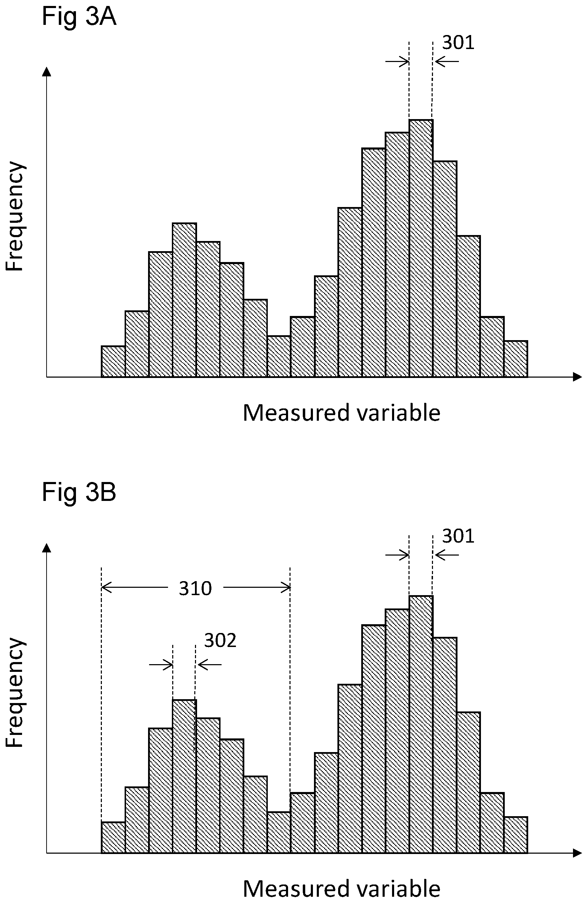

An example of such an analysis is illustrated in Figure 3A, which shows a histogram for the values of a measured variable for angiography data corresponding to a region of interest. In the example shown, the determination process identifies a sub-range 301 in which the greatest number of values fall.

A further example of such an analysis is illustrated in Figure 3B, which shows a histogram of the same data as Figure 3A. In the example shown in Figure 3B, the determination process is constrained so as to consider only sub-ranges falling within a predetermined interval 310 that is associated with the contrast agent. Thus, sub-ranges outside of this interval 310 may be ignored, even though a greater number of values may fall within those sub-ranges. Accordingly, the determination process illustrated in Figure 3B identifies sub-range 302, even though sub-range 301 corresponds to the peak for the overall distribution of the measured variable.

The sub-range identified by such a process may correspond to the regional characteristic. For instance, the midpoint of the thus-identified sub-range may be assigned as the regional characteristic. The regional characteristic may then be used during a later visualization process, which is part of block 140, for example to enhance the visibility of vessels in the region of interest in comparison to surrounding tissues.

In other examples, the determination process may comprise analysing spatial patterns within the subset of the angiography data corresponding to the region of interest. For example, the determination process may comprise identifying vessel-like forms within the region of interest. For instance, simple geometric forms, such as cylinders and truncated cones, may be identified. In other examples, centre lines for vessels may be identified. In still other examples, a segmentation process may be used, which may for example utilize a neural network and, in particular examples, a deep learning neural network.

In general, approaches that analyse spatial patterns in the angiographic data may provide, for each data element, a value indicating the likelihood that the data element corresponds to a vessel. To determine the regional characteristic(s), the determination process may analyse only data elements having at least a certain likelihood of being vessels. In some examples, the determination process might analyse the relative frequency with which, for such data elements, the values of the measured variable fall into various sub-ranges. Such an analysis may be carried out in generally the same manner as described above with reference to Figure 3A or Figure 3B. As before, the thus-identified sub-range may correspond to the regional characteristic. For instance, the midpoint of the thus-identified sub-range may be assigned as the regional characteristic.

In some examples, the regional characteristic(s) produced by the determination process of block 130 may define a range of values for the measured variable. For example, where a particular value for the measured variable is assigned as the regional characteristic (e.g. where the midpoint of the sub-range with highest frequency is assigned as the regional characteristic), this may implicitly define a range of predefined width that is centred about the value. In another example, two regional characteristics may be produced by the determination process of block 130 that define, respectively, upper and lower bounds for a range.

At block 140 of the angiographic data processing method 100, a visualization process is performed by the data processing system. The visualization process uses the regional characteristic(s) to generate image data that is representative of a view of the region of interest. Providing such a view assists the user in analysing the angiographic data. The visualization process may, for example, be a 3D rendering process, such as a ray-tracing process.

In some examples, the view may show parts of the imaged volume 200 other than the region of interest. However, the visualization process may still use the regional characteristic to visualize such parts of the imaged volume 200 outside of the region of interest. This may provide the user with a consistent visual representation of the angiographic data.

In other examples, the view generated by the visualization process may be limited (or substantially limited) to the region of interest. For example, the field of view for the view generated by the visualization process may be limited such that only the region of interest is visible. Additionally, or alternatively, only data elements corresponding to the region of interest may be rendered, and thus data elements corresponding to locations behind the region of interest (as seen from the viewing direction) may not be rendered.

As noted above, the visualization process may use the regional characteristic(s), as determined in block 130, to enhance the visibility of vessels in the view of the region of interest. For example, the visualization process may render the vessels with greater opacity (in particular, the vessel silhouettes may be rendered with greater opacity) and/or with different (e.g. more prominent) colouring than the surrounding tissues.

Because the regional characteristic(s) are indicative of the concentration of contrast agent within the particular region of interest, and because the contrast agent may be expected to be present largely within the vessels of the region of interest, the regional characteristics) may be considered to effectively indicate the data elements for the region of interest that correspond to vessels. As a result, the visualization process may use the regional characteristic(s) to enhance the visibility of vessels in the view of the region of interest. The regional characteristic(s) might therefore be described as defining a "window", with data within this window being rendered more prominently.

In particular examples, the visualization process may use the at least one regional characteristic that was determined in block 130 to determine, for at least some of the data elements, respective values for a visualization parameter. Such a visualization parameter may, for instance, govern how a given data element is visualized/rendered. The visualization parameter may, for example, be opacity or colour. Hence, in cases where the regional characteristic(s) define a range of values for the measured variable, each data element with a value falling within the range might be assigned an opacity value greater than a threshold value and/or might be assigned a particular colour value.

In some examples, the visualization process comprises a direct volume rendering process. In a direct volume rendering process, every data element in the angiographic data may be rendered as a solid element having one or more visualization parameter values, such as opacity and/or a colour values, that are determined with the aid of a transfer function. Thus, the transfer function may be considered to map the value(s) of the measured variable(s) for each data element to visualization parameter values for the solid element corresponding to that data element. As may be appreciated, in such examples, the regional characteristic(s) may be used as parameters for the transfer function. Thus, when the user-designated region of interest changes, causing corresponding changes to the regional characteristic(s), corresponding changes will be made to the transfer function.

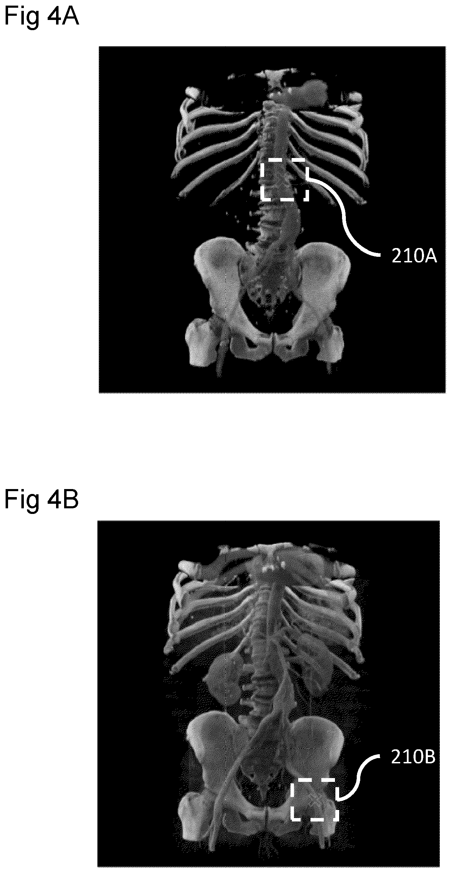

The action of the visualization process is illustrated with the aid of Figures 4A and 4B. For the purposes of illustration, each figure shows the effect of the visualization process when applied to an entire imaged volume. The same angiographic data was used to generate the visualizations shown in both Figures 4A and 4B. However, the region of interest 210A, 210B designated by the user is different in each case.

Figure 4A shows a situation where the region of interest 210A is located in the chest region of a patient. As shown, this results in the vessels of the chest being shown more prominently than the surrounding tissues - in particular the rib bones. By contrast, vessels in the hip region are rendered as being transparent.

Figure 4B shows a situation where the region of interest 210B is located in the hip region of a patient. As shown, this results in the vessels of the hip region being shown more prominently than in Figure 4A - in particular they are shown as opaque in Figure 4B, whereas in Figure 4A they are transparent.

As noted above, in particular examples, the data indicating the region of interest may comprise gaze direction data (e.g. data defining the direction in which the user's eyes are looking) and/or user view direction data (e.g. data defining the direction in which a user's head is facing). It is considered that, in such cases, the visualization of the angiographic data is particularly intelligible to the user. In particular, it is expected that users will intuitively understand the adjustments to the visualization process as their view direction changes.

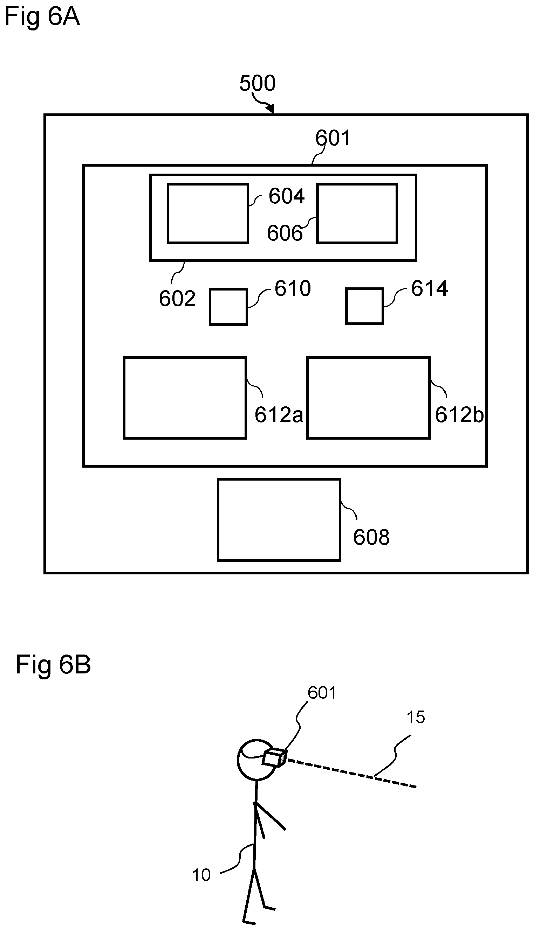

In many embodiments, the image data generated by the visualization process is representative of a 2D image. However, embodiments are also envisaged in which the image data is representative of two (or possibly more) 2D images, in particular where it is intended to provide stereoscopic 3D vision to a user, as in the system shown in Figure 6A and 6B.

The method 100 may include additional steps not shown in Figure 1. In particular, the method may, for example, include a step of displaying the view of the region of interest to the user, for example using one or more screens. As will be described with reference to Figures 6A and 6B below, in some examples, such screens may be provided within a headset 601 worn by the user 10.

It should be appreciated that, in some examples, the steps of method 100 may be repeated (e.g. with predefined frequency) and/or method 100 may run continually. Hence, where the data indicating the user-designated region of interest signify a change in the region of interest, block 130 may determine updated regional characteristic(s) corresponding to the new region of interest. The visualization of block 140 may then generate new image data that represents a view of the new region of interest. In this way, the method may react on-the-fly to changes in the user-designated region of interest.

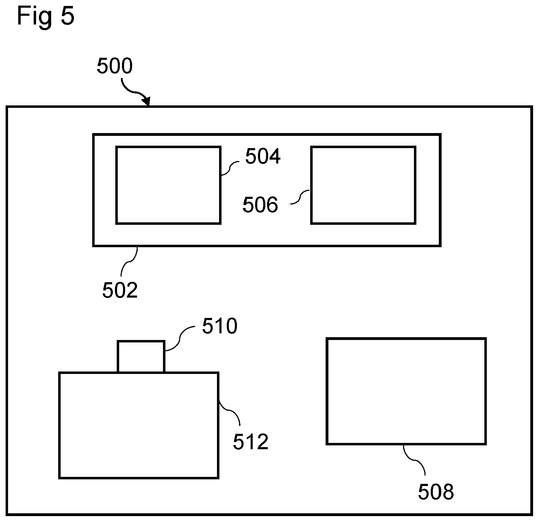

Attention is now directed to Figure 5, which schematically illustrates a system 500 for processing angiographic data. In the example shown, the system 500 comprises a computing device in the form of a computer 502. The computer 502 comprises one or more processors 504 and a memory 506. The memory 506 may be in the form of a computer readable storage medium. The memory 506 has stored on it instructions that, when executed by the one or more processors 504, cause the one or more processors to perform the angiographic data processing method described above.

The instructions may be stored on the memory 506 when the system 500 is supplied to a user. Alternatively, the instructions may be provided to the user thereafter, for example in the form of a computer program product, e.g. by means of a computer readable storage medium such as a compact disk (CD), a digital versatile disk (DVD), hard disk drive, solid state drive, a flash memory device and the like. Alternatively, the instructions may be downloaded onto the storage medium 506 via a data communication network (e.g. the worldwide web).

In cases where the method carried out by the one or more processors 504 involves one or more neural networks, such neural networks may be stored on memory 506. As with the instructions stored on memory 506, the neural networks may be stored on the memory 506 when the system 500 is supplied to a user, or may be provided thereafter (e.g. in the form of a computer program product), whether by means of a computer readable storage medium, or by means of downloading the neural networks via a data communication network.

Particularly in cases where the method involves one or more neural networks, the one or more processors 504 may comprise one or more Graphics Processing Units (GPUs), for example, or other types of processors. The use of GPUs may optimize the system 500 for making use of neural networks. This is because, as will be appreciated, a GPU can handle a large number of threads at the same time.

As illustrated in Figure 5, the system 500 may, in some examples, comprise one or more displays 512 for displaying to a user the view of the region of interest that was generated by the visualization process. In some examples, such as the system shown in Figures 6A and 6B, such displays may be provided within a headset 601 that is worn by the user. However, in other examples, such as that shown in Figure 5, such displays 512 may simply be screens visible to the user. In the specific example shown in Figure 5, a gaze tracking device 510 is associated with (e.g. mounted on) the display screen 512 and generates data indicating the user's gaze direction. Such a gaze tracking device 510 may, for example include one or more cameras configured to image the eyes of the user. The gaze direction data generated by the gaze tracking device 510 may be communicated to the one or more processors 504 and may provide part of, or all of the data indicating a user-designated region of interest.

As also illustrated in Figure 5, the system 500 may additionally comprise an imaging apparatus 508 configured to acquire the medical image data. For example, the system 500 may include an X-ray or MRI imaging apparatus.

In some examples, the system 500 may comprise an input interface such as a mouse, a keyboard (or respective connection interfaces for connecting same), a touch screen interface and the like. A user of the system 500 may use the input interface to input information into the system 500.

In a particular example, the user may use the input interface to manually adjust the regional characteristic used by the visualization process. Furthermore, where the angiographic data processing method makes use of a determination neural network, such user-provided adjustments to the regional characteristic may be used to further train the determination network.

Attention is now directed to Figures 6A and 6B, which schematically illustrate a further example of a system 600 for processing angiographic data. In the particular example shown, the system 600 comprises a headset 601, which is worn by a user 10.

As is apparent from Figure 6A, the system of Figures 6A and 6B may include similar components to the system of Figure 5. In particular, the system 600 includes a computing device 602, which comprises one or more processors 604 and a memory 606. The memory 606, which may be in the form of a computer readable storage medium, has stored on it instructions that, when executed by the one or more processors 604, cause the one or more processors to perform the angiographic data processing method described above.

As illustrated in Figure 6A, the headset 601 may, in some examples, comprise one or more orientation sensors 614, which generate data representative of the headset's orientation and thus the user's view direction 15. The headset orientation data generated by the orientation sensor(s) 614 may be communicated to the one or more processors 604 and may provide part of, or all of the data indicating a user-designated region of interest. In addition, the headset orientation data may be used by the visualization process so that the view of the region of interest is rendered from a viewing direction corresponding to the current headset orientation. In some examples, the headset might include further sensors, such as accelerometers, that are operable to sense translations of the headset. The output from such sensors may likewise be used in the visualization process so that the view of the region of interest is rendered from a viewing direction that moves (translates) corresponding to the current headset position.

As further illustrated in Figure 6A, the headset 601 may additionally (or alternatively) comprise a gaze tracking device 610. The gaze direction data generated by the gaze tracking device 610 may be communicated to the one or more processors 604 and may provide part of the data indicating a user-designated region of interest.

In many cases, as illustrated in Figure 6A, the headset 601 may comprise one or more displays 612a, 612b. In the particular example shown in Figure 6A, two displays 612a, 612b are provided, one for each of the user's eyes, for example so as to provide stereoscopic 3D vision to the user.

In the particular example shown in Figure 6A, the computing device 602 is provided on-board the headset 601, for example being an integrated component thereof. However, in other examples, the computing device 602 could be provided separately from the headset 601, with the computing device 602 and headset 601 having a data connection that enables the computing device 602 to transmit image data to the headset 601 for display using the headset displays 612a, 612b.

In some examples, the system 600 may comprise an input interface such as a mouse, a keyboard (or respective connection interfaces for connecting same), a touch screen interface and the like. Such an input interface may have similar functionality to the user interface described above with reference to system 500.

While the invention has been illustrated and described in detail in the context of specific examples, the invention is not limited to the disclosed examples. Other variations can be deducted by those skilled in the art without leaving the scope of protection of the claimed invention.

In summary, disclosed is a method, a computer readable storage medium and a system for processing angiography data. Angiography data is received at a data processing system. The angiography data is collected by scanning an imaged volume of a patient into whose vessels a contrast agent has been introduced. Data indicating a user-designated region of interest within the imaged volume is received at the data processing system. A determination process is performed at the data processing system. The determination process acts on the angiography data to determine at least one regional characteristic, which is indicative of the concentration of the contrast agent within the region of interest. A visualization process is performed at the data processing system. The visualization process acts on the angiography data and the at least one regional characteristic, to generate image data representative of a view of the region of interest.

## Claims

1. A method (100) for processing angiography data, the method comprising:
receiving (110), at a data processing system, angiography data, which is collected by scanning an imaged volume of a patient (1) into whose vessels a contrast agent has been introduced;
receiving (120), at the data processing system, data indicating a user-designated region of interest (210) within the imaged volume (200);
performing (130), at the data processing system, a determination process, which acts on the angiography data to determine at least one regional characteristic, which is indicative of the concentration of the contrast agent within the region of interest (210);
performing (140), at the data processing system, a visualization process, which acts on the angiography data and the at least one regional characteristic, to generate image data representative of a view of the region of interest (210).

2. The method (100) of claim 1, wherein the data indicating the region of interest (210) comprise user gaze direction data.

3. The method (100) of claim 1 or claim 2, wherein the determination process (130) comprises analysing spatial patterns within a subset of the angiography data corresponding to the region of interest (210).

4. The method (100) of claim 3, wherein the determination process (130) comprises identifying vessel-like forms within the region of interest (210).

5. The method (100) of any preceding claim, wherein the data processing system is a neural network system and the determination process comprises a segmentation process, which is carried out by a neural network.

6. The method (100) of any preceding claim, wherein the angiography data comprise a plurality of data elements, each of which corresponds to one of a plurality of locations that are distributed over the imaging volume (200), and is representative of a value for a measured variable at the location in question.

7. The method (100) of claim 6, wherein the visualization process (140) comprises determining, for at least some of the data elements, respective values for a visualization parameter, using the regional characteristic.

8. The method (100) of claim 7, wherein the visualization parameter is opacity.

9. The method (100) of any one of claims 6 to 8, wherein the at least one regional characteristic defines a range of values for said measured variable.

10. A computer readable storage medium, storing instructions that, when executed by a processor, cause the processor to:
receive (110) angiography data, which is collected by scanning an imaged volume of a patient into whose vessels a contrast agent has been introduced;
receive (120) data indicating a user-designated region of interest (210) within the imaged volume (200);
perform (130) a determination process, which acts on the angiography data to determine at least one regional characteristic, which is indicative of the concentration of the contrast agent within the region of interest (210); and
perform (140) a visualization process, which acts on the angiography data and the at least one regional characteristic, to generate image data representative of a view of the region of interest (210).

11. A system (500, 600) for processing medical image data, comprising:
one or more processors (504, 604); and
a memory (506, 606) storing instructions that, when executed by the one or more processors (504, 604), cause the one or more processors to:
receive (110) angiography data, which is collected by scanning an imaged volume of a patient into whose vessels a contrast agent has been introduced;
receive (120) data indicating a user-designated region of interest (210) within the imaged volume (200);
perform (130) a determination process, which acts on the angiography data to determine at least one regional characteristic, which is indicative of the concentration of the contrast agent within the region of interest (210); and
perform (140) a visualization process, which acts on the angiography data and the at least one regional characteristic, to generate image data representative of a view of the region of interest (210).

12. The system (500, 600) of claim 11, comprising one or more displays (512, 612) for displaying said view to the user (10) .

13. The system of claim 11 or claim 12, comprising a headset (601), which comprises one or more orientation sensors (614), which generate data representative of an orientation of the headset,
wherein the data indicating the region of interest (210) comprise the orientation data generated by the one or more orientation sensors (614).

14. The system (500, 600) of claim 12 and claim 13, wherein said headset (601) comprises said one or more displays (612a, 612b) .

15. The system (500, 600) of any one of claims 11-14, comprising a gaze tracking device (510, 610), which generates data representative of a gaze direction for the user (10),
wherein the data indicating the region of interest (210) comprise the data generated by the gaze tracking device.

16. The system (500, 600) of any one of claims 11-15, comprising an imaging apparatus (508, 608) configured to provide the angiographic data.

17. The system (500, 600) of any one of claims 11-16, comprising an input interface for allowing the user (10) to manually adjust the at least one regional characteristic.

18. The method (100) of any one of claims 1-9, the computer readable storage medium of claim 10, or the system (500, 600) of any one of claims 11-17, wherein the angiography data is computed tomography angiography data.
